# DEMANDE DE BREVET EUROPEEN

(11) **EP 1 665 947 A1**
(43) Date de publication de la demande: **07.06.2006**
(21) Numéro de dépôt: 04028457.2
(22) Date de dépôt: 01.12.2004
(51) Int. Cl.: A44C 5/00, A44C 5/14, A61B 5/02

(54) **Elément d'indication de la tension de serrage d'une bande, notamment d'un bracelet de dispositif portable**

(71) Demandeur: ETA SA Manufacture Horlogère Suisse, 2540 Grenchen (CH)
(72) Inventeur: Robin, Brice, 2300 La Chaux-de-Fonds (CH); Wyssbrod, Baptist, 2560 Nidau (CH); Karapatis, Nakis, 2520 La Neuveville (CH)
(74) Mandataire: Vigand, Philippe

(57) **Abrégé**

La présente invention concerne une bande (1) de serrage du type sangle ou bracelet de dispositif portable (40), par exemple, comportant des moyens (5) de fermeture et de réglage de sa longueur. La bande de serrage comporte un élément (6, 42) d'indication de sa tension de serrage présentant une apparence visuelle spécifique et identifiable par son utilisateur dans le but d'indiquer à ce dernier lorsque la tension de serrage de la bande est suffisante pour une application donnée. La bande de serrage de la présente invention est particulièrement intéressante lorsqu'elle est mise en oeuvre sous la forme d'un bracelet pour pulsomètre portable au poignet comprenant des moyens de mesure de type optique. Dans cette application, une valeur de la tension suffisante pour garantir une bonne précision des mesures est prédéfinie, les caractéristiques de l'élément d'indication étant adaptées à cette valeur pour permettre d'indiquer au porteur qu'il l'a atteinte au moment d'ajuster le bracelet sur son poignet.

## Description

### DOMAINE TECHNIQUE

La présente invention concerne une bande de serrage comportant au moins une première et une seconde extrémités libres ainsi que, d'une part, des moyens de fermeture et de réglage de sa longueur.

De manière générale, une telle bande peut être mise en oeuvre sous la forme d'une sangle de maintien pour rendre solidaires un certain nombre d'objets entre eux, ou encore sous la forme d'une ceinture, par exemple.

De manière préférée, la présente invention concerne une telle bande lorsqu'elle est mise en oeuvre en tant que bracelet pour rendre un dispositif portable, comme par exemple une montre, solidaire du bras d'un porteur.

### ARRIÈRE-PLAN TECHNOLOGIQUE

Beaucoup de bracelets de dispositifs portables, notamment de montres, connus de l'état de la technique répondent à la définition ci-dessus.

Toutefois, dans le cas de l'utilisation d'une montre à fonction multiples, par exemple un pulsomètre utilisant des capteurs placés dans le fond de la boîte de montre et devant venir en contact étroit avec le poignet du porteur, il est nécessaire de garantir un bon serrage lors de la prise de mesure, ce serrage devant être maintenu pendant toute l'opération du pulsomètre . En effet, comme de tels appareils réalisent généralement diverses opérations telles que des moyennes de mesures à partir des valeurs de pouls mesurées pendant un temps donné, il est nécessaire d'assurer qu'aucune interruption de mesure ne vienne fausser la moyenne pour que la valeur affichée soit bien représentative de l'effort fourni pendant ledit temps donné.

Le serrage du bracelet sur le poignet du porteur doit donc pouvoir être réglé précisément pour placer la montre dans de bonnes conditions de mesure du pouls et maintenu dans ces conditions de réglage même dans des conditions de travail difficiles comme lors d'une course à pied pendant laquelle le bracelet est soumis à des chocs et des vibrations importants.

Le brevet US 6,529,754 B2, délivré le 4 mars 2003 au nom de Seiko Epson Corporation, prévoit un pulsomètre comportant un bracelet permettant de répondre au problème mentionné ci-dessus, du fait que ce bracelet comporte des zones d'élasticité variable suivant sa périphérie. A titre d'exemple, ce document prévoit un mode de réalisation particulier selon lequel le bracelet comporte deux parties principales rigides ainsi que deux maillons solidaires des parties rigides et présentant une valeur d'élasticité plus importante que celle des parties rigides.

Grâce à ces maillons supplémentaires d'élasticité élevée, le boîtier reste plaqué sur le poignet de l'utilisateur même lors de mouvements importants de son poignet, en particulier en rotation.

Toutefois, cette solution ne prévoit pas de moyens pour indiquer au porteur du pulsomètre si le bracelet est suffisamment serré pour assurer un bon plaquage du boîtier sur son poignet et donc une bonne précision des mesures. Il est en effet prévu que le porteur du pulsomètre décrit dans ce brevet serre son bracelet de manière à ne pas ressentir une sensation de compression trop importante, similaire à la sensation ressentie lors du porter d'une montre conventionnelle.

### RÉSUMÉ DE L'INVENTION

Un but de la présente invention est de pallier les inconvénients susmentionnés de l'art antérieur en proposant une bande de serrage fournissant à son utilisateur des informations relatives à la tension de serrage de la bande, en particulier en lui indiquant lorsque la tension de serrage est adaptée aux conditions correspondantes d'utilisation, de manière intuitive et lisible.

Dans ce but, l'invention prévoit notamment une bande de serrage du type mentionné plus haut, caractérisée par le fait qu'elle comporte, d'autre part, un élément d'indication de la tension de serrage apte à présenter une configuration visuelle spécifique et identifiable par l'utilisateur pour une valeur de la tension appliquée à la bande sensiblement égale à une valeur prédéfinie. L'élément d'indication comprend préférablement une partie déformable de manière élastique délimitée par une première et une seconde extrémités rigides.

De manière préférée, la bande selon la présente invention comporte une portion principale tandis que l'élément indicateur de la tension de serrage est un maillon dont les extrémités rigides présentent des moyens d'attache à la portion principale. Préférablement, la partie déformable du maillon comprend au moins un trou qui peut être déplacé ou déformé en fonction de la tension de serrage de la bande pour laisser apparaître un repère lorsque la tension de serrage atteint une valeur suffisante. De manière alternative, on peut prévoir que le trou présente une première forme au repos et qu'il se déforme lorsque la tension de serrage augmente jusqu'à présenter une seconde forme prédéfinie, lorsque la tension de serrage atteint ou dépasse la valeur souhaitée.

Dans un mode de réalisation préféré, les moyens de fermeture et de réglage de la longueur de la bande comprennent des moyens de réglage de la longueur de manière continue.

Grâce à ces caractéristiques, lorsque la bande selon la présente invention est par exemple mise en oeuvre sous la forme d'une sangle de maintien et qu'elle est utilisée pour rendre solidaires des objets fragiles ou déformables, sa tension de serrage peut être ajustée précisément pour garantir un bon maintien des objets sans qu'aucun d'entre eux ne soit endommagé.

Par ailleurs, suivant une application préférée de la présente invention, la bande est mise en oeuvre sous la forme d'un bracelet pour permettre à un utilisateur de porter un dispositif du type montre multifonction au poignet. L'intérêt d'un bracelet répondant aux caractéristiques susmentionnées devient évident lorsqu'il est mis en oeuvre sur une montre comportant notamment une fonction de mesure de données physiologiques de son porteur, plus précisément une fonction de pulsomètre dont les mesures sont effectuées par voie optique. Dans ce cas, en effet, les caractéristiques énumérées ci-dessus permettent au porteur de la montre d'ajuster précisément la tension de serrage du bracelet pour garantir une bonne qualité des mesures effectuées, sans ressentir toutefois de compression inconfortable au niveau de son poignet.

### BRÈVE DESCRIPTION DES DESSINS

D'autres caractéristiques et avantages de la présente invention apparaîtront plus clairement à la lecture de la description détaillée qui suit, faite en référence aux dessins annexés donnés à titre d'exemples non limitatifs et dans lesquels:
- la figure 1 représente une vue schématique en perspective d'une bande de serrage selon un premier mode de réalisation préféré de la présente invention;
- la figure 2a représente une vue en coupe d'un détail de la bande représentée sur la figure 1 dans une première configuration;
- la figure 2b représente une vue en coupe similaire à la vue de la figure 2a, dans une configuration différente de la bande, et
- la figure 3 représente une vue schématique en perspective d'une partie d'un dispositif portable pourvu d'une bande selon un second mode de réalisation préféré de la présente invention.

### DESCRIPTION DÉTAILLÉE

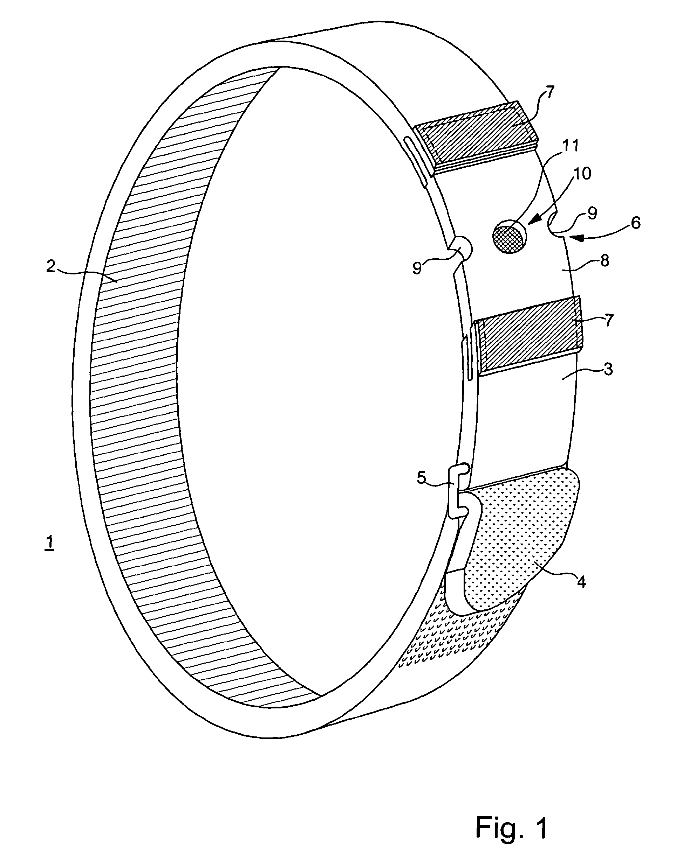

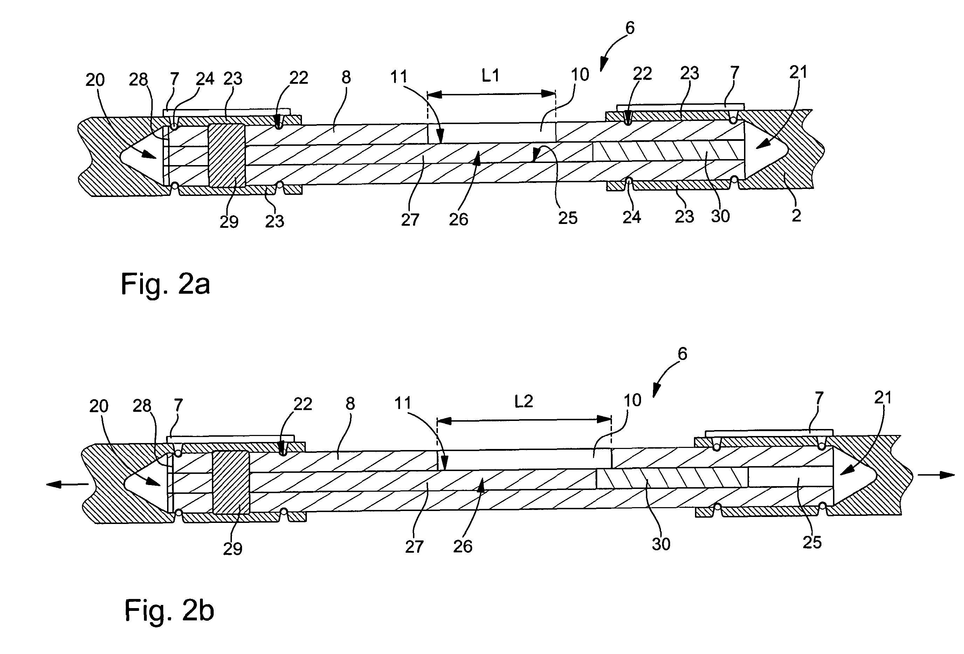

Les figures 1, 2a et 2b représentent une bande de serrage selon un premier mode de réalisation général de la présente invention.

La figure 1 montre la bande de serrage 1 lorsqu'elle est fermée sur elle-même. La bande 1 comporte une première portion principale 2 présentant deux extrémités libres 3 et 4, reliées l'une à l'autre par des moyens de fermeture et de réglage de la longueur de la bande.

Les moyens de fermeture et de réglage de la longueur de la bande peuvent être mis en oeuvre de plusieurs manières connues de l'état de la technique. On a représenté ici, à titre indicatif, des moyens préférés sous la forme d'une boucle 5 de laquelle est solidaire une première extrémité 3 de la bande 1, par exemple par une couture. La seconde extrémité 4 passe au travers de la boucle 5 et se replie sur elle-même, des moyens étant prévus pour maintenir fermement la seconde extrémité contre la portion principale, c'est-à-dire figer la longueur de la bande 1 dans cette position. A titre d'exemple, tel que schématisé sur la figure 1, on peut prévoir un système de boucles et crochets complémentaires, tels que ceux connus sous la dénomination commerciale "Velcro", répartis de manière adaptée sur l'extrémité 4 de la bande ainsi que sur la région de la portion principale qui lui est adjacente.

L'homme du métier ne rencontrera pas de difficulté particulière pour adapter tout autre système de fermeture connu adapté à la mise en oeuvre de la présente invention. Toutefois, on peut noter que, concernant les moyens de fermeture et de réglage de la longueur de la bande 1, des moyens particuliers permettant d'effectuer un réglage de manière continue de la longueur de la bande sont préférés pour la mise en oeuvre de la présente invention. On peut mentionner, à titre indicatif, un exemple supplémentaire d'un tel système de fermeture et de réglage, à savoir une boucle de fermeture du type de celles utilisées sur les ceintures d'avion, solidaire d'une première extrémité de la bande et bloquant la seconde extrémité libre de la bande par compression. Bien entendu, la mise en oeuvre de la présente invention reste avantageuse par rapport aux dispositifs connus de l'art antérieur même dans les cas où les moyens de fermeture et de réglage de la longueur de la bande ne fonctionnent pas de manière continue.

La portion principale 2 de la bande peut être réalisée en tout matériau permettant de la réaliser à la fois flexible et avec une faible élasticité, comme par exemple du cuir, un textile naturel ou synthétique non déformable, ou encore des matériaux plastiques à faible élasticité comme des polyuréthanes, par exemple.

D'autre part, la bande 1 comporte une portion supplémentaire 6 plus courte que la portion principale 2. La disposition de la portion supplémentaire 6 suivant la circonférence de la bande 1 est indifférente de manière générale mais peut avoir une incidence dans certaines applications particulières. Ainsi, dans le cas du mode de réalisation représenté sur la figure 1, la portion supplémentaire 6 est représentée à distance de l'extrémité 3 de la bande.

Les moyens de connexion mis en oeuvre pour intégrer la portion supplémentaire 6 dans la circonférence de la portion principale 2 de la bande 1 sont plus clairement visibles sur les figures 2a et 2b et seront abordés en détail plus bas.

On a représenté à titre indicatif des éléments décoratifs 7 optionnels permettant de dissimuler les moyens de connexion de la portion supplémentaire 6 à la portion principale 2. Ces éléments décoratifs 7 peuvent être réalisés en tout matériau adapté, comme par exemple un plastique conventionnel, et peuvent être mis à profit pour apposer la marque de fabrique du fabricant de la bande 1.

La portion supplémentaire 6 qui remplit une fonction d'indicateur de la tension de serrage de la bande comprend à cet effet une partie centrale 8 présentant une élasticité élevée, notamment dans la direction longitudinale de la bande 1. En outre, la partie centrale 8 présente une forme particulière visant à favoriser sa déformation dans la direction longitudinale de la bande 1, à savoir des évidements 9 latéraux.

Par ailleurs, la partie centrale 8 est pourvue d'un trou 10 en son centre, dont la fonction sera expliquée ci-dessous en relation avec la description détaillée des figures 2a et 2b. De manière préférée non limitative, le trou 10 est circulaire dans sa configuration au repos, tel que représenté sur la figure 1 et laisse apparaître une surface 11 de texture prédéfinie, comme par exemple une couleur prédéfinie.

La figure 2a représente un détail en coupe de la bande 1 dans sa configuration représentée sur la figure 1, la coupe étant prise dans un plan coupant la bande en son milieu et s'étendant dans la direction longitudinale de cette dernière.

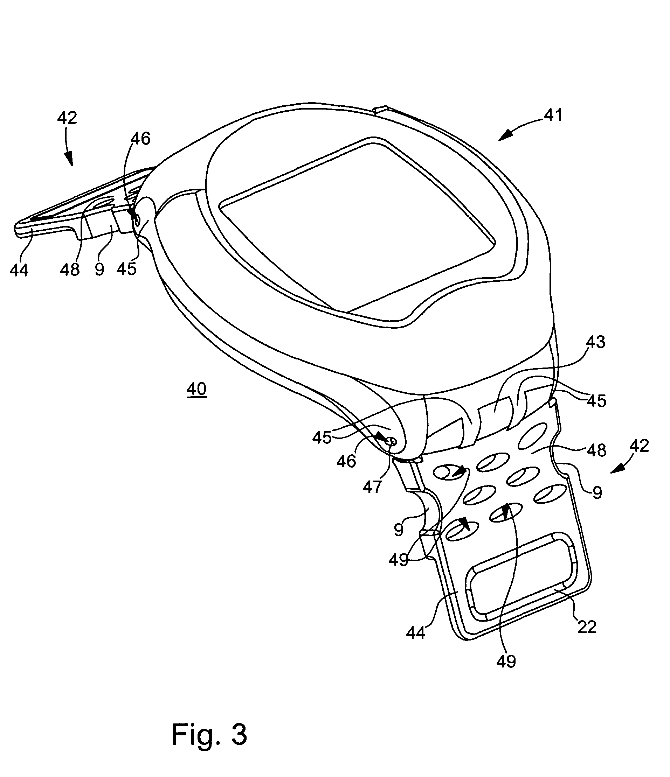

Suivant le mode de réalisation préféré représenté, la portion supplémentaire 6 comporte deux extrémités rigides 20 et 21, de part et d'autre de la portion centrale 8 élastique. Chacune de ces extrémités 20, 21 comporte une gorge 22, de forme sensiblement rectangulaire (visible sur la figure 3) et destinée à coopérer avec des moyens d'attache de la portion principale 2. Chacune des parties de la portion principale 2 reliées à la portion supplémentaire 6 est divisée en deux extrémités 23 symétriques par rapport au plan moyen de la bande et comportant chacune un petit cadre 24, réalisé en plastique rigide ou en métal, qui peut éventuellement être cousu sur ou dans l'extrémité 23 correspondante. Chaque cadre 24 présente une forme et des dimensions adaptées pour permettre son insertion dans la gorge 22 de l'extrémité rigide 20, 21 correspondante de la portion supplémentaire. De manière préférée, à chaque extrémité rigide 20, 21 de la portion supplémentaire 6, les deux extrémités 23 symétriques sont cousues entre-elles au moins sur les côtés et éventuellement au travers de l'extrémité rigide 20, 21 correspondante, de telle manière que chacun des cadres 24 est fermement maintenu dans sa gorge 22. Une telle structure permet de garantir une connexion efficace et solide entre les portions principale et supplémentaire de la bande 1. En outre, on peut noter que de tels moyens de connexion permettent également de renforcer la rigidité des deux extrémités 20, 21.

Les éléments décoratifs 7 recouvrent les moyens de connexion des portions principale et supplémentaire, à savoir les cadres 24 ainsi que les coutures, du côté externe de la bande, c'est-à-dire de son côté qui est apparent lorsqu'elle est utilisée.

Selon le mode de réalisation représenté à titre indicatif, la portion supplémentaire 6 présente un creux central 25 de forme parallélépipédique dans lequel est logée une plaquette 26, de forme complémentaire. La plaquette 26 est préférablement réalisée en un matériau rigide et est éventuellement agencée dans la portion supplémentaire 6 par surmoulage. Un premier côté 27 de la plaquette 26 est solidaire de l'extrémité rigide 20 du fait de l'arrangement d'une partie des coutures 28, rendant les deux extrémités symétriques 23 solidaires l'une de l'autre, et du fait de l'agencement d'un pied 29 traversant les deux extrémités symétriques 23 ainsi que la plaquette 26. Bien entendu, la présente invention ne se limite pas aux moyens de fixation du premier côté 27 de la plaquette 26 qui viennent d'être décrits, qui peuvent être mis en oeuvre indépendamment l'un de l'autre, ou encore qui peuvent alternativement mettre en oeuvre un collage conventionnel du premier côté 27 de la plaquette avec l'extrémité rigide 20 de la portion supplémentaire 6.

On a schématisé deux régions successives, dans la plaquette, dans la direction longitudinale de la bande, une première région correspondant au premier côté 27, puis une seconde région 30, s'étendant depuis la fin de la première région jusqu'à l'extrémité de la plaquette située du côté de l'extrémité rigide 21. La première région présente une partie apparente 11 au travers du trou 10 de la partie centrale 8. De manière préférée, les deux régions 27, 30 présentent des couleurs différentes facilement distinguables l'une par rapport à l'autre, comme par exemple du rouge et du vert.

On constate sur la figure 2a que la seconde région 30 n'est pas visible au travers du trou 10, ce dernier présentant une longueur au repos dans la direction longitudinale notée L1. Ainsi, dans cette configuration de la bande, l'utilisateur perçoit un disque rouge au travers du trou 10.

Lorsque la bande 1 est tendue, tel que représenté sur la figure 2b, dans le but de maintenir des objets ensemble par exemple, la tension subie par la portion supplémentaire augmente, entraînant une déformation de sa partie centrale 8 élastique. La déformation de la partie centrale 8 entraîne notamment une déformation, en particulier un étirement, du trou 10 de manière que ce dernier présente alors une longueur notée L2 dans la direction longitudinale de la bande 1, L2 étant supérieure à L1.

Il est en outre apparent de la figure 2b qu'en plus de se déformer, le trou 10 se déplace pour suivre la déformation plus générale de la partie centrale 8, notamment jusqu'à rendre apparente une partie de la seconde région 30 de la plaquette 26. On peut noter en effet que, du fait de la structure décrite précédemment pour la portion supplémentaire 6, la plaquette 26 ne subit pas de déplacement ou de déformation en réponse à une augmentation de la tension de serrage de la bande 1. De manière préférée, l'utilisateur voit apparaître à ce stade une coloration verte à côté de la coloration rouge initiale, dans le but de lui indiquer que la tension de serrage de la bande a atteint une valeur suffisante dans le cadre de l'utilisation en cours de la bande 1.

Par conséquent, pour une application prédéfinie de la bande 1, l'homme du métier sait quelle tension de serrage optimale doit présenter la bande lorsqu'elle est tendue et peut ainsi prévoir la déformation nécessaire du trou 10 pour laisser apparaître la seconde région 30 de la plaquette 26 et indiquer à l'utilisateur que la tension est suffisante. De ce fait, lorsque la bande sert à maintenir ensemble des objets fragiles, ces caractéristiques peuvent être ajustées à la fabrication pour garantir l'indication à l'utilisateur de la tension nécessaire et suffisante pour parvenir à ce but, sans endommager les objets.

Bien entendu, l'indication de la tension de serrage selon la présente invention ne se limite pas à l'utilisation d'un élément changeant de couleur tel que cela vient d'être décrit. On peut prévoir simplement l'apparition d'une couleur ou encore un changement de texture au sens le plus large, le principe de base consistant au moins à obtenir une apparence identifiable par l'utilisateur lorsque la valeur de la tension de serrage requise est atteinte.

La figure 3 représente un mode de réalisation préféré de la bande selon la présente invention, lorsqu'elle est mise en oeuvre en tant que bracelet pour permettre de porter au poignet un dispositif portable 40, du type montre bracelet. La bande selon le présent mode de réalisation n'a été représentée que partiellement pour rendre certains détails de sa structure apparents.

La portion principale (non représentée) de la bande est ici divisée en deux brins similaires entre lesquels est arrangé le boîtier 41 d'un dispositif électronique portable 40.

Selon une variante préférée de mise en oeuvre, le dispositif électronique portable est un pulsomètre comprenant des moyens de mesure du pouls de son porteur ainsi que des circuits électroniques de traitement des mesures et de commande de moyens d'affichage permettant d'afficher les résultats obtenus. De tels dispositifs électroniques sont connus de l'art antérieur et ne seront pas décrits plus en détail, dans la mesure où ils ne contribuent pas directement à la présente invention. A titre indicatif, l'homme du métier pourra se reporter à l'enseignement du brevet américain mentionné au titre d'art antérieur pour consulter un exemple de mise en oeuvre d'un dispositif électronique présentant une fonction de mesure des pulsations cardiaques de son porteur, en particulier par l'utilisation de moyens de mesure de type optique.

Concernant les moyens de fermeture et de réglage de la bande, ils ne sont plus représentés sur la figure 3, dans la mesure où ils peuvent être mis en oeuvre de manière similaire à ce qui a été décrit en relation avec la description détaillée de la figure 1. De manière alternative, on peut prévoir de mettre en oeuvre un dispositif conventionnel de fermeture de bracelet, du type boucle et ardillon coopérant avec des trous, combiné à un dispositif spécifique de réglage de la longueur du bracelet, du type de celui décrit dans la demande de brevet européen No. 04009671.1, déposée le 23 avril 2004 au nom de The Swatch Group Management Services AG. Bien entendu, l'homme du métier pourra mettre en oeuvre tout autre système de fermeture et de réglage connu, adapté à la mise en oeuvre de la bande décrite ici sans sortir du cadre de la présente invention.

On a représenté sur la figure 3 une variante préférée de réalisation selon laquelle la bande comprend deux éléments 42 d'indication de la tension de serrage de la bande identiques, disposés de part et d'autre du boîtier 41.

Chacun de ces éléments 42 comporte deux extrémités rigides 43 et 44, dont l'une 43 est connectée au boîtier 41. Dans ce but, le boîtier comporte quatre cornes 45 au travers desquelles est ménagé un trou cylindrique 46, l'extrémité rigide 43 présentant une forme complémentaire adaptée pour s'emboîter entre les cornes 45. En outre, l'extrémité rigide 43 comprend également un trou cylindrique (non visible) ménagé de manière à être aligné avec le trou 46, une barrette 47 permettant de rendre les cornes et l'extrémité rigide 43 solidaires.

La seconde extrémité rigide 44 est similaire à l'extrémité rigide 21 décrite en relation avec la description détaillée de la figure 2a du précédent mode de réalisation. L'extrémité rigide 44 comprend en effet une gorge 22 de forme sensiblement rectangulaire et destinée à loger un cadre rigide solidaire du brin de bracelet correspondant (non représentés). Le brin est alors préférablement cousu pour être rendu solidaire de l'élément ou maillon indicateur 42, tel que décrit plus haut.

La partie centrale 48 de chaque élément indicateur 42 est déformable de manière élastique du fait d'une élasticité de cette partie plus importante que celles des extrémités rigides 43, 44. L'élasticité de la partie centrale 48 est due, notamment, à sa structure particulière. On constate effectivement que la partie centrale 48 comprend des évidements latéraux 9, identiques à ceux décrits en relation avec la figure 1, destinés à faciliter des déformations longitudinales de l'élément indicateur 42 en réponse à une augmentation de la tension subie entre ses deux extrémités rigides.

En outre, la partie centrale 48 comporte une pluralité de trous 49 traversants et présentant chacun une forme sensiblement elliptique. Ainsi, lorsque la tension exercée sur le bracelet augmente, la partie centrale 48 de chaque maillon 42 se déforme provoquant une déformation des trous 49.

En fonction de certains paramètres, tel que la forme précise des trous 49 et les propriétés élastiques de la partie centrale 48, que l'homme du métier peut ajuster sans difficulté, il est possible de définir quasiment n'importe quel type d'apparence prédéfinie, identifiable par l'utilisateur, que l'on souhaite obtenir pour une valeur prédéfinie de la tension de serrage du bracelet.

En particulier, sur la base des trous 49 représentés sur la figure 3, on peut définir les propriétés de chacun des maillons 42 de sorte que, pour une valeur de la tension de serrage prédéfinie, les trous subissent une déformation pour présenter une forme circulaire ou encore une forme de losange.

L'homme du métier ne rencontrera pas de difficulté particulière pour ajuster le choix du matériau des maillons 42 et la forme des trous 49 pour obtenir une apparence prédéfinie identifiable par l'utilisateur lorsque la tension de serrage correspond à une valeur optimale pour l'application concemée.

Alternativement, on peut prévoir une structure comprenant un ou plusieurs trous similaires aux trous 49 et arrangés de telle manière qu'ils subissent une déformation, lorsque la tension de serrage de la bande augmente, pour former une inscription lorsque la valeur de la tension de serrage est sensiblement égale à la valeur souhaitée.

Une telle solution permet avantageusement au fabricant de la bande ou du dispositif d'apposer sa marque de fabrique de façon originale sur son produit.

Le dispositif représenté sur la figure 3 correspond à une application préférée de la bande selon la présente invention qui ne peut être considérée comme étant limitative. En effet, le bracelet tel qu'il est décrit ci-dessus présente un avantage particulier lorsqu'il est mis en oeuvre sur un dispositif présentant une fonction de pulsomètre dont les mesures sont effectuées à l'aide de moyens de mesure de type optique. Ces moyens de mesure comprennent généralement un émetteur de faisceaux lumineux ainsi qu'un récepteur d'au moins une partie des faisceaux lumineux émis après qu'ils se sont propagés dans l'organisme du porteur. De ce fait, les résultats des mesures sont sensibles à des sources lumineuses externes au dispositif, notamment à la lumière ambiante. C'est pourquoi il est important que le boîtier du dispositif portable soit correctement plaqué contre la peau du porteur. Dans ce but, la tension de serrage doit présenter une valeur minimale à partir de laquelle la lumière environnante ne peut plus perturber les mesures. Ainsi, le bracelet tel qu'il est partiellement représenté sur la figure 3 permet non seulement de plaquer correctement le boîtier contre le poignet du porteur, même en cas de mouvements de ce dernier, grâce à l'élasticité des maillons 42 directement reliés au boîtier, mais également d'indiquer au porteur lorsque la tension de serrage est suffisante pour assurer une bonne fiabilité des mesures. Cette dernière caractéristique permet avantageusement au porteur d'ajuster précisément la tension de serrage dans une mesure telle que le confort du dispositif n'est pas affecté.

Selon une variante de réalisation, on peut prévoir une combinaison des éléments d'indication de la tension de serrage de la bande selon les deux modes de réalisation décrits ci-dessus. On peut en effet prévoir qu'un élément d'indication de la tension de serrage tel que représenté sur la figure 3 comporte un creux similaire au creux 25 de la figure 2a, dans lequel est logée une plaquette similaire à la plaquette 26 et servant de référentiel. Dans ce cas, la plaquette comprend, sur sa face 11, des motifs correspondant à la position et à la forme des trous 49, lorsque ces derniers sont déformés en réponse à la valeur souhaitée de la tension de serrage de la bande. La présente variante de réalisation permet avantageusement au porteur d'identifier plus facilement la situation dans laquelle les trous 49 ont atteint la forme souhaitée que dans le cas de la réalisation de la figure 3, mais avec une construction sensiblement plus complexe que cette dernière.

Selon une variante de réalisation supplémentaire, il est possible de se passer des trous 49 en prévoyant que la partie centrale 48 est pleine et que les trous 49 sont remplacés par de simples motifs agencés sur la surface apparente de la partie centrale 48. Les motifs peuvent être formés dans la masse de la partie centrale élastique ou imprimés sur sa surface par tout procédé adapté. Il est alors possible d'adapter les propriétés mécaniques de la partie centrale élastique pour réaliser de tels motifs qui, à partir d'une forme quelconque dans la position de repos de la partie centrale, présentent toute forme prédéfinie identifiable par l'utilisateur lorsque la tension de serrage de la bande atteint une valeur prédéfinie. De manière similaire à ce qui a été décrit plus haut, on peut prévoir, par exemple, que les motifs obtenus sont des disques, des losanges ou encore forment une inscription prédéfinie identifiable.

Dans le cadre de la présente variante de réalisation, on peut également prévoir que les motifs sont portés par un élément support additionnel solidaire de la partie centrale élastique, comme par exemple, de manière non limitative, par une fine plaquette en silicone surmoulée sur la partie centrale élastique ou inversement.

La description qui précède correspond à des modes de réalisation préférés de l'invention et ne saurait en aucun cas être considérée comme limitative, en ce qui concerne plus particulièrement la structure décrite pour les moyens de fermeture et de réglage de la longueur de la bande ou encore les moyens de connexion des différents éléments constitutifs de la bande. L'homme du métier ne rencontrera pas de difficulté particulière pour adapter les caractéristiques particulières de la bande telle que décrite à ses propres besoins, en termes de matériaux, de forme ou de dimensions sans sortir du cadre de la présente invention.

## Revendications

1. Bande de serrage (1) comportant au moins une première et une seconde extrémités libres (3, 4), des moyens (5) de fermeture et de réglage de sa longueur, **caractérisée en ce qu'**elle comporte en outre un élément (6, 42) d'indication de la tension de serrage apte à présenter une configuration visuelle spécifique identifiable par un utilisateur pour une valeur de la tension appliquée à ladite bande sensiblement égale à une valeur prédéfinie.

2. Bande selon la revendication 1, **caractérisée en ce que** ledit élément (6, 42) d'indication de la tension de serrage comprend au moins une partie (8, 48) déformable de manière élastique.

3. Bande selon la revendication 2, **caractérisée en ce que** ladite partie (8, 48) déformable est délimitée par une première (20, 43) et une seconde (21, 44) extrémités rigides dudit élément (6, 42) d'indication de la tension de serrage.

4. Bande selon la revendication 3, **caractérisée en ce qu'**elle comporte au moins une portion principale (2), ledit élément étant un maillon (6, 42) dont lesdites extrémités rigides (20, 21, 44) présentent des moyens d'attaches (22) pour coopérer avec des moyens d'attache (24) adaptés de ladite portion principale (2).

5. Bande selon l'une quelconque des revendications 2 à 4, **caractérisée en ce que** ladite partie déformable (48) comporte au moins un trou (49) dont la section présente une forme dépendant de la valeur de la tension appliquée à ladite bande (1).

6. Bande selon la revendication 5, **caractérisée en ce que** ledit trou (49) présente une section circulaire, en forme de losange ou définit une inscription lorsque la valeur de la tension appliquée à ladite bande est sensiblement égale à ladite valeur prédéfinie.

7. Bande selon la revendication 5 ou 6, **caractérisée en ce que** ledit élément (42) comprend en outre des moyens (26) de référence solidaires de l'une (20) desdites extrémités et portant au moins un motif agencé de manière à être situé en regard dudit trou et à présenter la même forme que ledit trou lorsque la valeur de la tension appliquée à ladite bande est sensiblement égale à ladite valeur prédéfinie.

8. Bande selon l'une quelconque des revendications 2 à 4, **caractérisée en ce que** ladite partie déformable (48) comporte au moins un motif présentant une forme dépendant de la valeur de la tension appliquée à ladite bande (1).

9. Bande selon la revendication 8, **caractérisée en ce que** ledit motif présente une forme circulaire, de losange ou définit une inscription lorsque la valeur de la tension appliquée à ladite bande est sensiblement égale à ladite valeur prédéfinie.

10. Bande selon la revendication 3 ou 4, **caractérisée en ce que** ladite partie déformable (8) comporte au moins un trou (10) dont la position par rapport à l'une (20) au moins desdites extrémités rigides dépend de la valeur de la tension de serrage de ladite bande (1), ledit élément (6) d'indication comportant en outre des moyens (26, 27, 30) de référence solidaires de ladite extrémité (20) et situés à une distance de cette dernière telle qu'ils présentent une apparence visuelle spécifique prédéfinie pour une valeur de la tension appliquée à ladite bande sensiblement égale à une valeur prédéfinie.

11. Bande selon la revendication 10, **caractérisée en ce que** lesdits moyens de référence comprennent deux portions (27, 30) de couleurs respectives différentes situées dans le prolongement l'une de l'autre suivant la direction longitudinale de ladite bande.

12. Bande selon l'une quelconque des revendications précédentes, **caractérisée en ce que** lesdits moyens (5) de fermeture et de réglage de la longueur de ladite bande comprennent des moyens de réglage de ladite longueur de manière continue.

13. Dispositif portable (40) comportant un boîtier (41) et une bande (1) selon l'une quelconque des revendications précédentes remplissant la fonction d'un bracelet, une première desdites extrémités libres de ladite bande comportant des moyens pour coopérer avec des premiers moyens adaptés d'un premier côté dudit boîtier, la seconde desdites extrémités étant adaptée pour coopérer avec lesdits moyens de fermeture et de réglage de la longueur, ces derniers comportant des moyens pour coopérer avec des seconds moyens adaptés d'un second côté dudit boîtier.

14. Dispositif portable selon la revendication 13, dans lequel ladite bande comprend un élément d'indication de la tension de serrage présentant la forme d'un maillon (42) dont une première (43) desdites extrémités rigides porte des moyens d'attache pour coopérer avec des moyens (45, 46, 47) adaptés dudit boîtier (41).

15. Dispositif portable (40) comportant un boîtier (41) et une bande (1) selon l'une quelconque des revendications 1 à 12 remplissant la fonction d'un bracelet et présentant deux extrémités libres (3, 4), ladite bande comportant au moins deux brins agencés de part et d'autre dudit boîtier et portant chacun l'une desdites extrémités libres, au moins l'un desdits deux brins comportant un élément (42) d'indication de la tension de serrage de ladite bande.

16. Dispositif portable selon la revendication 15, lesdites extrémités libres étant reliées aux dits moyens de fermeture et de réglage de la longueur de ladite bande, **caractérisé en ce qu'**au moins un élément (42) d'indication de la tension de serrage de ladite bande est agencé entre l'une desdites extrémités libres et ledit boîtier (41).

17. Dispositif portable selon la revendication 16, **caractérisé en ce que** ledit élément (42) d'indication de la tension de serrage de ladite bande est relié directement au boîtier (41).

18. Dispositif portable selon la revendication 17, **caractérisé en ce qu'**un second élément (42) d'indication de la tension de serrage de ladite bande est relié directement au boîtier.

19. Dispositif selon l'une quelconque des revendications 13 à 18, **caractérisé en ce qu'**il comporte des moyens de mesure de données physiologiques de son porteur, lesdits moyens de mesure étant intégrés au dit boîtier (41).

20. Dispositif portable selon la revendication 19, **caractérisé en ce qu'**il comporte une fonction de pulsomètre, des moyens de mesure optiques étant disposés dans une face dudit boîtier destinée à être agencée contre le bras dudit utilisateur.
